# Europäisches Patentamt

## European Patent Office

## Office européen des brevets

(19)

(11) Publication number: **0 070 132**
**B1**

(12) **EUROPEAN PATENT SPECIFICATION**

(45) Date of publication of patent specification: **18.06.86**

(51) Int. Cl.⁴: **A 61 B 17/36**

(21) Application number: **82303504.3**

(22) Date of filing: **02.07.82**

(54) Adaptor extension mechanism for a laser scalpel.

(30) Priority: **07.07.81 JP 100952/81 u**

(43) Date of publication of application:
**19.01.83 Bulletin 83/03**

(45) Publication of the grant of the patent:
**18.06.86 Bulletin 86/25**

(84) Designated Contracting States:
**DE FR GB SE**

(56) References cited:
**EP-A-0 033 958**
**US-A-3 865 113**

(73) Proprietor: **SUMITOMO ELECTRIC INDUSTRIES LIMITED**
**No. 15, Kitahama 5-chome Higashi-ku**
**Osaka-shi Osaka 541 (JP)**

(72) Inventor: **Sunago, Katsuyoshi**
**c/o Osaka Works of Sumitomo Electric Ind. Ltd.**
**1-3, Shimaya 1-chome Konohana-ku Oska (JP)**
Inventor: **Iwamoto, Tomio**
**c/o Osaka Works of Sumitomo Electric Ind. Ltd.**
**1-3, Shimaya 1-chome Konohana-ku Osaka (JP)**

(74) Representative: **George, Sidney Arthur et al**
**GILL JENNINGS & EVERY 53-64 Chancery Lane**
**London WC2A 1HN (GB)**

## Description

This invention relates to an adaptor extension mechanism for a laser scalpel.

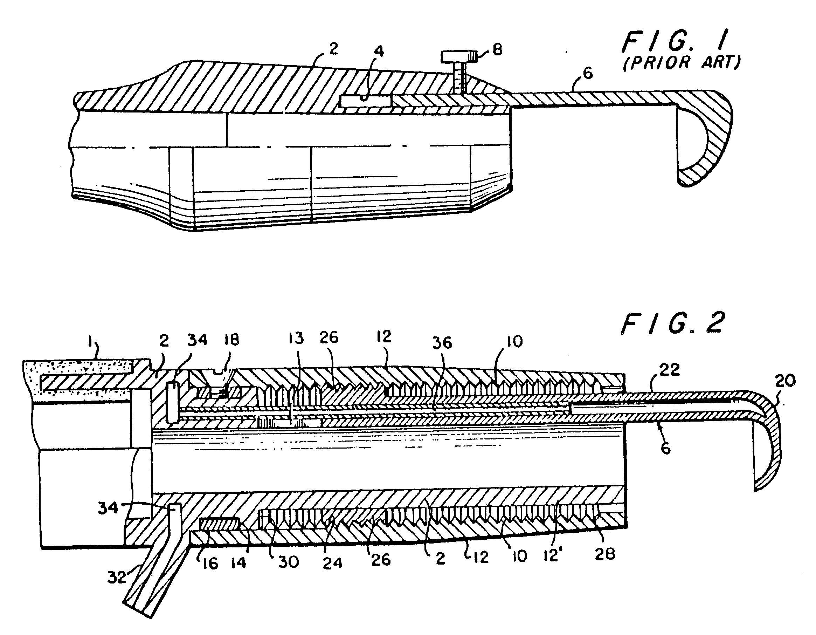

Heretofore, an adaptor extension mechanism for a laser scalpel has been known. As shown in Figure 1 of the accompanying drawing, such a mechanism comprises a main body 2 provided with a longitudinal groove 4, and an adaptor end portion 6 which is slidably mounted in the groove 4. The portion 6 is held in a desired position relative to the main body 2 by means of a cap screw 8. In use of the device, the portion 6 is so positioned that its head lies under the focusing part of the laser scalpel in order to interrupt the laser beam, thereby preventing cutting of tissue beneath the head of the portion 6. The depth of the tissue to be cut varies from stage to stage of a surgical operation. Therefore, the adaptor portion 6 must be slidable in the axial direction of the main body of the adaptor. However, the known extension mechanism has a defect in that it requires considerable time to position the portion 6, because it involves loosening the screw 8, sliding the portion 6 in or out and then retightening the screw 78. This prolongs the operating time.

Our European Patent Application EP—A— 0 033 958, which is part of the prior art under Article 54(3) and (4) of the European Patent Convention only, discloses another adaptor extension mechanism for a laser scalpel, in which an adapter end portion is slidable in the axial direction of the main body of the adaptor. An internally-threaded sleeve is mounted in a groove in the main body so that the sleeve is rotatable around the body. A threaded part of the adaptor end portion, positioned between the main body and the sleeve, engages with the internal thread of the sleeve, so that the adaptor end portion slides when the sleeve is rotated.

However, the mounting of the sleeve directly in the groove of the main body causes scratching and wearing of the groove, and early deformation or failure of the threaded part. Smooth rotation of the threaded sleeve and, hence, smooth sliding of the adaptor end portion cannot, therefore, be maintained. Furthermore, the assembly of the sleeve into the groove in the main body involves a difficult operation.

An object of the present invention is to provide an adaptor extension mechanism for a laser scalpel which allows smooth positional adjustment of the adaptor end portion, and which is easy to assemble.

According to the invention, there is provided an adaptor extension mechanism for a laser-powered scalpel, in which a longitudinally extending adaptor end portion is slidable in the axial direction of an elongate main body of the adaptor, the mechanism comprising an internally-threaded sleeve having a ring attached thereto by screw means, the sleeve being mounted for rotation around the main body by engagement of the ring in a circumferential groove in the external surface of the main body; and a threaded part of the adaptor end portion, positioned between the main body and the sleeve, which part engages with the internal thread of the sleeve so that the adaptor end portion is slidable by rotation of the sleeve.

The position of the adaptor end portion is thereby easily and speedily adjustable.

An embodiment of the invention will now be described, by way of example, with reference to the accompanying drawing, in which:

Figure 1 is a partial cross section showing the conventional adaptor extension mechanism as described above; and

Figure 2 is a partial cross section showing an extension mechanism according to the present invention.

Figure 2 shows the application of the present invention to a laser scalpel provided with an adaptor of a beam-receiving dish type. The beam-receiving dish interrupts the laser beam in order to prevent cutting of tissue beneath the dish. Cutting of tissue is therefore effected above the dish, as is known to those skilled in the art. An adaptor holding part 1 of the end portion of the laser scalpel is provided with a substantially cylindrical main adaptor body 2. A sleeve 12 having multiple threads 10 cut on its internal surface is mounted for rotation around the body 2.

The sleeve 12 is mounted on the main body 2 by forming a circumferential groove 14 in the external surface of the main body 2, mounting a ring 16 rotatably in the groove 14, and connecting the sleeve 12 to the ring 16 by means of a screw 18. A longitudinal guide groove 13 is formed in a circumferential wall 12' of the main body 2, extending from the part which holds the ring 16 to the end of the body. An adaptor end portion i.e. a tube 22 with a beam-receiving dish 20 at its end, is integrally provided with ring portion 26 having an external thread 24 which engages with an internal thread 10 of the sleeve 12. The tube 22 is slidably positioned in the guide groove 13. The ring portion 26 is located between the main body 2 of the adaptor and the sleeve 12. Accordingly, rotation of the sleeve 12 leads to inward or outward movement of the beam-receiving dish 20, the movement being limited by engagement of the ring 26 with stopping surfaces 28 and 30 on the main adaptor body 2.

In addition, feed water, i.e. a physiological solution of sodium chloride, is introduced into the beam-receiving dish 20 from a pipe 32 mounted in the main body 2, via an annular groove 34 in the main body 2 and a hollow feed water needle 36. The needle 36 is fixed to the main body 2 in the guide groove 13 so that the tube 22 receives the needle 36.

In use of the mechanism according to the present invention, it is necessary only to rotate the sleeve 12 in order to extend or retract the beam-receiving dish 20. Hence, the position of the beam-receiving dish 20 can be easily and speedily set, thereby shortening the operation time. Further, although an adaptor of the beam-receiving dish type is described in the above preferred embodiment, an adaptor extension mechanism according to the present invention can be used for adaptors of other types in which extension of the end part of the adaptor is required.

## Claims

1. An adaptor extension mechanism for a laser-powered scalpel, in which a longitudinally extending adaptor end portion (6) is slidable in the axial direction of an elongate main body (2) of the adaptor, the mechanism comprising an internally-threaded sleeve (12) having a ring (16) attached thereto by screw means (18), the sleeve being mounted for rotation around the main body by engagement of the ring in a circumferential groove in the external surface of the main body; and a threaded part (24) of the adaptor end portion, positioned between the main body and the sleeve, which part engages with the internal thread (10) of the sleeve so that the adaptor end portion is slidable by rotation of the sleeve.

2. A mechanism as claimed in claim 1, characterised in that the adaptor end portion (6) comprises a hollow rod (22) having a beam-receiving dish (20) on the end thereof.

3. A mechanism as claimed in claim 2, characterised in that the main body (2) includes a longitudinal guide groove (13) in a circumferential wall (12') of the main body, and the hollow rod (22) is slidably positioned in the guide groove.

4. A mechanism as claimed in claim 2 or claim 3, characterised in that the main body (2) includes a feed water groove (34) and a hollow feed water needle (36) fixed to the main body, the needle extending into the hollow rod for feeding water to the beam-receiving dish (20).

5. A mechanism as claimed in claim 1, characterised in that the adaptor end portion (6) includes a ring (26) and a hollow rod (22) integrally formed with the ring, the threaded ring portion (24) being formed on the ring.

6. A mechanism as claimed in claim 5, characterized in that the main body (2) includes stopping surfaces (28, 30) engageable with the ring (26) to limit sliding movement of the adaptor end portion.

## Patentansprüche

1. Verlängerungsmechanismus für ein Anpaßstück eines laserbetriebenen Skalpells, bei welchem ein sich längs erstreckendes Anpaßendteil (6) in axialer Richtung eines länglichen Hauptkörpers (2) des Anpaßstückes verschiebbar ist, mit einer ein Innengewinde aufweisenden Hülse (12), die einen mit einem Schraubenmittel (18) an ihr befestigten Ring (16) aufweist und um den Hauptkörper durch Eingriff des Rings in eine umlaufende Nut in der äußeren Oberfläche des Hauptkörpers drehbar gelagert ist, und einem Gewindeteil (24) des Anpaßendteils, der zwischen dem Hauptkörper und der Hülse liegt und in das Innengewinde (10) der Hülse eingreift, so daß der Anpaßendteil durch Drehung der Hülse verschiebbar ist.

2. Mechanismus nach Anspruch 1, dadurch gekennzeichnet, daß der Anpaßendteil (6) einen hohlen Stab (22) aufweist, der an seinem Ende einen strahlaufnehmenden Fänger (20) besitzt.

3. Mechanismus nach Anspruch 2, dadurch gekennzeichnet, daß der Hauptkörper (2) eine längliche Führungsnut (13) in einer Umfangswand (12') des Hauptkörpers umfaßt und der hohle Stab (22) verschiebbar in der Führungsnut angeordnet ist.

4. Mechanismus nach Anspruch 2 oder 3, dadurch gekennzeichnet, daß der Hauptkörper (2) eine Speisewasserrinne (34) sowie eine hohle Speisewassernadel (36), die am Hauptkörper befestigt ist und in den hohlen Stab ragt, um Wasser zum Strahl aufnehmenden Fänger (20) zu führen, umfaßt.

5. Mechanismus nach Anspruch 1, dadurch gekennzeichnet, daß der Anpaßendteil (6) einen Ring (26) und einen hohlen Stab (22), der aus einem Stück mit dem Ring besteht, aufweist und der Gewindeteil (24) am Ring angeformt ist.

6. Mechanismus nach Anspruch 5, dadurch gekennzeichnet, daß der Hauptkörper (2) Anschlagflächen (28, 30) besitzt, die mit dem Ring (26) in Eingriff bringbar sind, zur Begrenzung der Verschiebebewegung des Anpaßendteils.

## Revendications

1. Mécanisme d'extension d'un élément de réglage pour un scalpel alimenté par laser, où une partie extrême de réglage (6) s'étendant longitudinalement et coulissant en direction axiale d'un corps principal allongé (2) de l'élément de réglage, le mécanisme comprenant un manchon intérieurement fileté (12) ayant une bague (16) qui lui est attachée par un moyen fileté (18), le manchon étant monté pour une rotation autour du corps principal par engagement de la bague dans une gorge circonférentielle de la surface externe du corps principal; et une partie filetée (24) de la partie extrême de l'élément de réglage, placée entre le corps principal et le manchon, laquelle partie engage le filetage interne (10) du manchon de façon que la partie extrême de réglage soit coulissante par rotation du manchon.

2. Mécanisme selon la revendication 1 caractérisé en ce que la partie extrême de réglage (6) comprend une tige creuse (22) ayant un réflecteur paraboloïde (20) de réception du faisceau à son extrémité.

3. Mécanisme selon la revendication 2 caractérisé en ce que le corps principal (2) comprend une gorge longitudinale de guidage (13) dans une paroi circonférentielle (12') du corps principal, et la tige creuse (22) est placée coulissante dans la gorge de guidage.

4. Mécanisme selon la revendication 2 ou la revendication 3 caractérisé en ce que le corps principal (2) comprend une gorge (34) d'eau d'alimentation et une aiguille creuse (36) d'eau d'alimentation fixée au corps principal, l'aiguille s'étendant dans la tige creuse pour introduire l'eau vers le réflecteur paraboloïde (20) de réception du faisceau.

5. Mécanisme selon la revendication 1 caractérisé en ce que la partie extrême de l'élément de réglage (6) comprend une bague (26) et une tige creuse (22) intégralement formée avec la bague, la partie filetée (24) étant formée sur la bague.

6. Mécanisme selon la revendication 5 caractérisé en ce que le corps principal (2) comprend des surfaces d'arrêt (28, 30) pouvant venir en engagement avec la bague (26) pour limiter un mouvement coulissant de la partie extrême de l'élément de réglage.

*FIG. I*
*(PRIOR ART)*

*FIG. 2*

0 070 132